# EUROPEAN PATENT SPECIFICATION

(11) **EP 3 360 490 B1**
(45) Date of publication and mention of the grant of the patent: **01.07.2020**
(21) Application number: 17210311.1
(22) Date of filing: 22.12.2017
(51) Int. Cl.: A61B 17/29, A61B 1/005, A61B 17/00

(54) **FLEXIBLE TUBE**
FLEXIBLES ROHR
TUBE FLEXIBLE

(30) Priority: 14.02.2017 CN 201710079500
(43) Date of publication of application: 15.08.2018
(73) Proprietor: Zhuhai Pusheng Medical Science&Technology Co., Ltd, Zhuhai City Guangdong 519000 (CN)
(72) Inventor: ZENG, Guohua, Zhuhai City, Guangdong 519000 (CN); HUANG, Honghui, Zhuhai City, Guangdong 519000 (CN); WANG, Zhenwei, Zhuhai City, Guangdong 519000 (CN); WU, Jun, Zhuhai City, Guangdong 519000 (CN)
(74) Representative: Vitina, Maruta

(56) References cited:
- JP-A- 2015 047 453
- US-A- 5 807 241
- US-A1- 2012 143 175
- US-A1- 2013 226 151
- US-A1- 2014 180 009

## Description

### FIELD OF THE INVENTION

The present invention relates to technical field of flexible tubes, and more specially, relates to a flexible tube.

### BACKGROUND OF THE INVENTION

Existing flexible tube is manufactured by cutting centripetally through certain thickness of the tube and bending through a pair of symmetrical joints with larger outer diameter and smaller inner diameter formed by centripetal cutting, and the trapezoidal surface of the joints can ensure that the joints will not be disengaged in general. However, the strength of the flexible tube perpendicular to the bending direction is weak. If one pin is added to strengthen, that is, one pin of a segment of the flexible tube is inserted into the groove matched with adjacent segment, two pins are needed and the connection of the pin and the bending surface are suprafacial. The strength perpendicular to bending direction is strengthened with support of this pin, and this pin has no effect on the strength of bending direction. The strengthening effect is limited, when the pin is under slightly greater force, the pin is easily deformed, disengaged with groove and has no effect. The length of the pin can be extended for better reinforcing effect. However, overlong pin after bending will affect on internal space of the flexible tube.

There is known flexible tube [US 5 807 241 A], wherein the flexible tube comprises a plurality of structural units 1", 2", each of the structural units comprises a first connecting pipe and a second connecting pipe; wherein one end of the first connecting pipe is provided with a lost and a rectangular structure; one end of the second connecting pipe is provided with a round projection and a limiting slot; and the other end of the limiting slot is provided with a limiting structure; the round projection is located inside the slot, a top end of the second connecting pipe of adjacent structural unit, thereby forming a bendable flexible tube. However, this rectangle structure has similar operation as a pin described above, i.e. the strength perpendicular to bending direction is strengthened with support of this pin, and this pin has no effect on the strength of bending direction. The strengthening effect is limited, when the pin is under slightly greater force, the pin is easily deformed, disengaged with groove and has no effect.

The closest prior art is a flexible tube [US 2013/226151 A1] consisting of a number of structural units, each of said units comprises a first connecting pipe and a second connecting pipe, wherein one end the first connecting pipe is provided with a slot and a first projection; one end of the second connecting pipe is provided with a second projection and a limiting slot; a limiting structure is provided on both sides of one end of the limiting structure; second projection is located inside the slot, and the first projection is located inside the limiting slot, a top end of the second connecting pipe of the structural units is connected with a bottom end of the first connecting pipe of adjacent structural unit, thereby forming a bendable flexible tube. However, the flexible tube [US 2013/226151 A1] does not strengthen enough the strength of the flexible tube in the vertical bending direction.

### SUMMARY OF THE INVENTION

The present invention is to overcome the problems about strength difference of the flexible tube perpendicular to the bending direction without reinforcing structure, and great effect on the interior of the flexible tube with reinforcing structure by providing a flexible tube.

In order to solve the above technical problems, the present invention, as claimed in claim 1, provides the following technical solutions:
The present invention provides a flexible tube, comprising a plurality of structural units, each of the structural units comprises a first connecting pipe and a second connecting pipe, wherein a slot and a first projection in the form of a T-shaped structure are provided on one end of the first connecting pipe; a second projection and a limiting slot are provided on one end of the second connecting pipe; a limiting structure A is provided on both sides of one end of the limiting slot, and the other end of the limiting slot is provided with a limiting structure B; the second projection is located in the slot, and the T-shaped structure is located in the limiting slot; a top end of the second connecting pipe of the structural units is connected with a bottom end of the first connecting pipe of adjacent structural unit, thereby forming a curve tubular-shaped flexible tube successively connected by the plurality of structural units, wherein the T shaped structure has two flat side walls and is placed with the possibility of movement in surface-to-surface contact with flat inner walls of the limiting slot.

As a preferred embodiment of the present invention, the horizontal cross sections of the slot and the second projection are circular.

As a preferred embodiment of the present invention, the horizontal cross sections of the slot and the second projection are trapezoid.

The beneficial effect of the invention is that the flexible tube strengthens the vertical bending direction strength of the flexible tube by the T-shaped structure, the T-shaped structure is more effective than strip pin and has little effect on the internal structure of the flexible tube, and the strength of the bending direction of flexible tube has also been strengthened.

### BRIEF DESCRIPTION OF THE DRAWINGS

The accompanying drawings are intended to provide a further understanding of the invention and form a part of the specification, and are intended to be illustrative of the invention in conjunction with the embodiments of the invention and are not to be construed as limiting the invention. In the drawings:

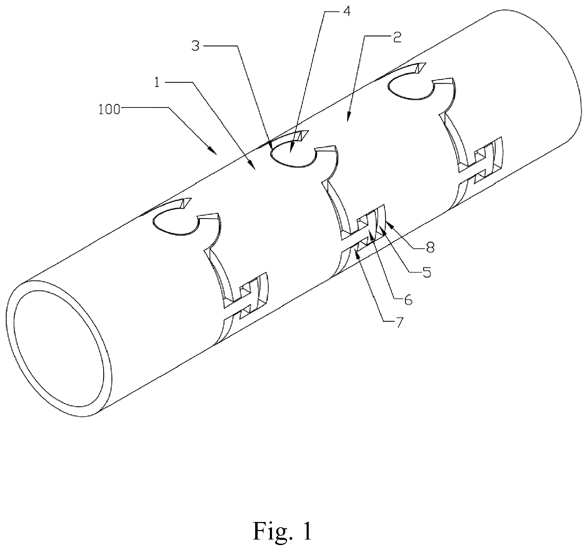

FIG. 1 is a structural view of the present invention.

In the figure, 1-first connecting pipe; 2-second connecting pipe; 3-slot; 4-second projection; 5-limiting slot; 6-T-shaped structure (first projection); 7-limiting structure A; 8-limiting structure B.

### DETAILED DESCRIPTION OF THE INVENTION

Preferred embodiments of the present invention are described with reference to the accompanying drawings, and it is to be understood that the preferred embodiments described herein are only for illustration purpose, and are not intended to limit the invention.

### Embodiment 1

As shown in FIG. 1, the present invention provides a flexible tube. The flexible tube includes a plurality of structural units 100, each of the structural units 100 comprises a first connecting pipe (1) and a second connecting pipe (2). A slot (3) and a T-shaped structure (6) are provided on one end of the first connecting pipe (1); a second projection (4) and a limiting slot (5) are provided on one end of the second connecting pipe (2); a limiting structure A (7) is provided on both sides of one end of the limiting slot (5), and the other end of the limiting slot (5) is provided with a limiting structure B (8); the second projection (4) is located in the slot (3), and the T-shaped structure (6) is located in the limiting slot (5); a top end of the second connecting pipe (2) of the structural units (100) is connected with a bottom end of the first connecting pipe (1) of adjacent structural unit (100), thereby forming a curve tubular-shaped flexible tube successively connected by the plurality of structural units (100).

The horizontal cross sections of the slot (3) and the second projection (4) are circular, which reduces the resistance when the flexible tube bends and makes it easier to bend. The horizontal cross sections of the slot (3) and the second projection (4) are trapezoid so that the first connecting pipe (1) and the second connecting pipe (2) are not easily to be disjointed when connecting with second projection (4) through the second projection (4).

Specific working principle: two flanks are provided on the original pin to form a T-shaped structure 6, other positions keep clearance. When the flexible tube bends to the limiting position, the T-shaped structure 6 stucks or pushes against the limiting position. First of all, the T-shaped structure 6 is a pin in nature, which can strengthen the strength perpendicular to the bending direction. Secondly, in the structure of the flexible tube with a pin, when the flexible tubes are broken perpendicularly to the bending direction, the pin is bent firstly, then the pin is disengaged, and finally the flexible tube is broken, and the T-shaped structure makes it difficult to disengage the pin, thereby increasing the strength of the pin in the direction perpendicular to the bend. And the T-shaped structure 6 is designed to push the limiting position when it is bent to the limiting position, and further bending of the flexible tubes can be prevented, and the strength of the bending direction can also be increased. When the flexible tube is flat, the T-shaped structure 6 has no effect. When the flexible tube is bent, the two T-shaped structures are respectively close to the limiting structure A 7 and the limiting structure B 8; when the flexible tube is bent to the limiting position, the T-shaped structure is pushed in place, and the strengthening effect begins to emerge.

The beneficial effect of the invention is that the flexible tube strengthens the vertical bending direction strength of the flexible tube by the T-shaped structure, compared with strip pin, the T-shaped structure is more effective and has little effect on the internal structure of the flexible tube, and the strength of the bending direction of flexible tube has also been strengthened.

It should be noted that the foregoing is only a preferred embodiment of the present invention and is not intended to limit the invention. Although the invention has been described in detail with reference to the foregoing embodiments, it will be apparent to those skilled in the art that the technical solutions described in the foregoing embodiments may be modified or equivalently replaced with some of the technical features therein. Any modifications, equivalent substitutions, improvements and the like within the principles of the invention are intended to be included within the scope of the present invention. The invention is defined by the following claims.

## Claims

1. A flexible tube, comprising: a plurality of structural units (100), each of the structural units (100) comprises a first connecting pipe (1) and a second connecting pipe (2);
wherein one end of the first connecting pipe (1) is provided with a slot (3) and a first projection (6); one end of the second connecting pipe (2) is provided with a second projection (4) and a limiting slot (5); a limiting structure A (7) is provided on both sides of one end of the limiting slot (5), and the other end of the limiting slot (5) is provided with a limiting structure B (8); the second projection (4) is located inside the slot (3), and the first projection (6) is located inside the limiting slot (5); a top end of the second connecting pipe (2) of the structural units (100) is connected with a bottom end of the first connecting pipe (1) of adjacent structural unit (100), thereby forming a curve tubular-shaped flexible tube successively connected by the plurality of structural units (100), **characterized in that** the first projection (6) is formed as a T-shaped structure with two flat side walls and placed with the possibility of movement in surface-to-surface contact with flat inner walls of the limiting slot (5).

2. The flexible tube according to claim 1, wherein a horizontal cross section of the slot (3) and the second projection (4) is circular.

3. The flexible tube according to claim 1, wherein a horizontal cross section of the slot (3) and the second projection (4) is trapezoid.

## Patentansprüche

1. Flexibles Rohr, umfassend: mehrere Struktureinheiten (100), wobei jeweilige der Struktureinheiten (100) ein erstes Verbindungsrohr (1) und ein zweites Verbindungsrohr (2) umfasst;
wobei ein Ende des ersten Verbindungsrohrs (1) mit einem Schlitz (3) und einem ersten Vorsprung (6) versehen ist; und wobei ein Ende des zweiten Verbindungsrohrs (2) mit einem zweiten Vorsprung (4) und einem Begrenzungsschlitz (5) versehen ist; und wobei eine Begrenzungsstruktur A (7) an beiden Seiten eines Endes des Begrenzungsschlitzes (5) vorgesehen ist und eine Begrenzungsstruktur B (8) an dem anderen Ende des Begrenzungsschlitzes (5) vorgesehen ist; und wobei der zweite Vorsprung (4) sich innerhalb des Schlitzes (3) befindet und der erste Vorsprung (6) sich innerhalb des Begrenzungsschlitzes (5) befindet; und wobei ein oberes Ende des zweiten Verbindungsrohrs (2) der Struktureinheiten (100) mit einem unteren Ende des ersten Verbindungsrohrs (1) der benachbarten Struktureinheit (100) verbunden ist, sodass ein krummes röhrenförmiges flexibles Rohr aus mehreren Struktureinheiten (100) nacheinander gebildet wird, **dadurch gekennzeichnet, dass** der erste Vorsprung (6) als T-förmige Struktur mit zwei flachen Seitenwänden ausgebildet ist und so angeordenet ist, dass der erste Vorsprung (6) die Möglichkeit einer Bewegung im Kontakt von der Oberfläche der flachen Seitenwänden mit der Oberfläche mit der flachen Innenwänden des Begrenzungsschlitzes (5) hat.

2. Flexibles Rohr nach Anspruch 1, **dadurch gekennzeichnet, dass** der horizontale Querschnitt des Schlitzes (3) und des zweiten Vorsprungs (4) kreisförmig ist.

3. Flexibles Rohr nach Anspruch 1, **dadurch gekennzeichnet, dass** der horizontale Querschnitt des Schlitzes (3) und des zweiten Vorsprungs (4) trapezförmig ist.

## Revendications

1. Un tube flexible, comprenant: une pluralité d'unités structurelles (100), chacune desdites unités structurelles (100) comprend un premier tuyau de raccordement (1) et un second tuyau de raccordement (2);
dans lequel une extrémité dudit premier tuyau de raccordement (1) est pourvue d'une fente (3) et d'une première saillie (6); une extrémité dudit deuxième tuyau de raccordement (2) est pourvue d'une seconde saillie (4) et d'une fente de limitation (5); une structure de limitation A (7) est disposée sur deux côtés d'une extrémité de ladite fente de limitation (5), et l'autre extrémité de ladite fente de limitation (5) est pourvue d'une structure de limitation B (8); ladite deuxième saillie (4) est située à l'intérieur de ladite fente (3), et ladite première saillie (6) est située à l'intérieur de ladite fente de limitation (5); une extrémité supérieure dudit deuxième tuyau de raccordement (2) desdites unités structurelles (100) est reliée à une extrémité de fond dudit premier tuyau de raccordement (1) de l'unité structurelle adjacente (100), ce qui permet de former un tube flexible de forme tubulaire courbe connecté successivement par ladite pluralité d'unités structurelles (100), **caractérisés en ce que** ladite première saillie (6) est formée comme une structure en forme de T avec deux parois latérales plates et placée avec la possibilité de mouvement en contact de surface à surface avec des parois intérieures plates de ladite fente de limitation (5).

2. Ledit tube flexible selon la revendication 1, dans lequel une section transversale horizontale de ladite fente (3) et une section transversale horizontale de ladite deuxième saillie (4) sont circulaires.

3. Ledit tube flexible selon la revendication 1, dans lequel une section transversale horizontale de ladite fente (3) et une section transversale horizontale de ladite deuxième saillie (4) sont trapézoïdales.
